# EUROPEAN PATENT APPLICATION

(11) **EP 3 811 788 A1**
(43) Date of publication of application: **28.04.2021**
(21) Application number: 20199545.3
(22) Date of filing: 01.10.2020
(51) Int. Cl.: A24B 13/00, B65B 9/04, B65D 75/32

(54) **PRODUCTION LINE FOR MANUFACTURING NICOTINE POUCHES**

(30) Priority: 23.10.2019 SE 1951203
(71) Applicant: Enorama Pharma AB, 211 34 Malmö (SE)
(72) Inventor: Rönngard, Mats-Olle, 216 18 Limhamn (SE)
(74) Representative: AWA Sweden AB

(57) **Abstract**

The invention relates to a method and a production line (1) for producing pouches (2). The product line (1) comprises a supply unit (3), a deformation unit (4), a dosing unit (5), a sealing unit (6) and a cutting unit (7).

## Description

### Technical field

The invention relates to a method and a production line for producing pouches.

### Background art

In recent years, with the recognition of the harmful effects of tobacco smoking, there have been numerous campaigns and programs by governmental agencies and various health groups and other interested organizations to disseminate information about the adverse health effects resulting from tobacco smoking. Moreover, and as a result of this recognition of the harmful effects, there have been many programs directed to attempts in reducing smoking incidence.

Nicotine is an organic compound and is the principal alkaloid of tobacco. Nicotine is the chief addictive ingredient in the tobacco used in cigarettes, cigars, snuff and the like. Nicotine is also an addictive drug, though, and smokers characteristically display a strong tendency to relapse after having successfully stopped smoking for a time. Nicotine is the world's second most used drug, after caffeine from coffee and tea.

The main problem with tobacco smoking is its enormous implications on health. Today it is estimated that smoking related diseases cause some 3-4 million deaths per year. In fact, excessive smoking is now recognized as one of the major health problems throughout the world. This grim consequence of tobacco smoking has urged many medical associations and health authorities to take very strong actions against the use of tobacco.

One way to reduce smoking is to provide nicotine in a form or manner other than by smoking and some products have been developed to fulfill this need. Nicotine containing formulations are currently the dominating treatments for tobacco dependence.

As an aid for those who are willing to stop smoking there are several ways and forms of nicotine replacement products available on the market. One successful product that is used as a smoking substitute and/or as a smoking cessation aid and which is based on nicotine is the nicotine containing pouch. The pouch is provided under the lip of a user and the nicotine is slowly released from the pouch upon exposure to moisture from the user.

Pouched smokeless tobacco products may be produced by measuring portions of the smokeless tobacco composition and inserting the portions into a nonwoven tube.

EP 3 330 191 A1 discloses an oral pouched snuff product and a method for its production. The method comprises the steps of supplying and advancing a tubular web of the mesh material enveloping a continuous feed of filling material in a direction of travel (L), forming transverse seals in said tubular web across said continuous feed of filling material by means of ultrasonic welding, said transverse seals extending in a transverse direction (T) being perpendicular to said direction of travel (L), and cutting the tubular web along the transverse direction, thereby forming a plurality of oral pouched snuff products.

A problem associated with the production method disclosed above as well as other production methods according to currently available prior art is that the current technique uses a narrow band of nonwoven material onto which the formulation is dosed continuously. The material is then formed into a cylinder and sealed horizontally and the pouches are formed by sealing and cutting off the cylinder into rectangular pouches. This results in a limited production capacity due to the fact that only one pouch at the time can be produced.

Another problem is that the formulation provided in the tubular web will render the sealing and cutting process more difficult since there is a risk that at least a portion of the formulation ends up in the sealing section.

### Summary of the invention

It is an objective of the present invention to mitigate, alleviate or eliminate one or more of the above-identified deficiencies in the art and disadvantages singly or in any combination and solve at least the above mentioned problems.

According to a first aspect of the invention, these and other objects, and/or advantages that will be apparent from the following description of embodiments, are achieved, in full or at least in part, by a method for producing pouches. The method comprises the steps of feeding a first layer of material into a deformation unit, deforming the first layer of material in the deformation unit, thereby creating recesses in the first layer of material, feeding the first layer of material into a dosing unit, providing a formulation in each recess of the first layer of material, feeding a second layer of material onto the first layer of material, sealing the first layer of material and the second layer of material together in at least one sealing section, and cutting the first layer of material and the second layer of material in the at least one sealing section, thereby forming at least one pouch.

This is advantageous in that the prooduction capacity can be increased without a theoretical end of the first and second layer of material. The wider the band and number of dosing units, the higher the capacity. This is compared with the industrial standard of today, 100-200 pouches/min.

The recesses created by the deformation unit will ascertain that there is no formulation between the layers of the packaging material during sealing. This gives a cleaner sealing with both better appearance and better durability. In turn, it also decreases the waste of formulation.

The formulation may be provided in the pouches is chosen from the group consisting of dry powder, a wet powder and a liquid solution dosed into an absorbing material.

The formulation may comprise nicotine.

The formulation may comprise synthetic or cannabis derived cannabinoids, cannabinoid acids, terpenoids, flavonoids and mixtures thereof.

The step of feeding the first layer of material into a deformation unit may comprise placing a first layer of material from a roll onto a moving band.

The step of sealing the first layer of material and the second layer of material together in at least one sealing section may comprise heat sealing or ultrasonic welding.

The step of cutting the first layer of material and the second layer of material in the at least one sealing section may comprise stamping out a plurality of pouches in one single operation.

The method may further comprise the step of feeding a protective layer onto the first layer of material and/or the second layer of material before sealing the first layer of material and the second layer of material together.

According to a second aspect of the invention, these and other objects are achieved, in full or at least in part, by a production line for producing pouches. The production line comprises a supply unit transporting a first layer of material along the production line, the first layer of material optionally being supplied from a roll of material, a deformation unit, through which the first layer of material travels, adapted to deform the first layer of material to create at least one recess therein, a dosing unit arranged downstream of the deformation unit, through which the first layer of material travels, adapted to provide a formulation in the at least one recess of the first layer of material, a sealing unit arranged downstream of the dosing unit, through which the first layer of material travels, and through which a second layer of material is supplied, the sealing unit being adapted to seal the first layer of material and the second layer of material together in at least one sealing section, and a cutting unit arranged downstream of the sealing unit, through which the sealed first layer and second layer of material travels, adapted to cut the first layer of material and the second layer of material in the at least one sealing section, thereby forming at least one pouch.

The production line may create any suitable shape of the pouch and is not restricted to one and the same package material. The equipment can utilize multiple layers of package material, adding different properties in each layer. These properties could be different flavors, API concentrations, buffers etc. They could possibly also be designed to form a protective surface, increasing durability of the product.

The supply unit may comprise a moving band onto which the first layer of material is placed to travel along the production line.

The deformation unit may comprise a first part having recesses, and a second part having protrusions adapted to engage with the recesses of the first part, the second part being arranged above the first part.

The dosing unit may comprise a reservoir for holding the formulation connected to a roll equipped with recesses adapted to receive a specified amount of the formulation, the roll being rotatably arranged in relation to the reservoir.

The dosing unit may comprise a reservoir for holding the formulation and being movable in a vertical direction in relation to the production line, the reservoir having openable bottom portion for releasing a specified amount of the formulation into a recess of the first layer of material.

The formulation provided in the pouches may be chosen from the group consisting of dry powder, a wet powder and a liquid solution dosed into an absorbing material.

The formulation may comprise nicotine or synthetic or cannabis derived cannabinoids, cannabinoid acids, terpenoids, flavonoids and mixtures thereof.

Effects and features of the second and third aspects of the present invention is largely analogous to those described above in connection with the first aspect the inventive concept. Embodiments mentioned in relation to the first aspect of the present invention are largely compatible with the further aspects of the invention.

Other objectives, features and advantages of the present invention will appear from the following detailed disclosure, from the attached claims, as well as from the drawings. It is noted that the invention relates to all possible combinations of features.

Generally, all terms used in the claims are to be interpreted according to their ordinary meaning in the technical field, unless explicitly defined otherwise herein. All references to "a/an/the [element, device, component, means, step, etc.]" are to be interpreted openly as referring to at least one instance of said element, device, component, means, step, etc., unless explicitly stated otherwise.

As used herein, the term "comprising" and variations of that term are not intended to exclude other additives, components, integers or steps.

### Brief description of the drawings

The above objects, as well as additional objects, features and advantages of the present invention, will be more fully appreciated by reference to the following illustrative and non-limiting detailed description of preferred embodiments of the present invention, when taken in conjunction with the accompanying drawings, wherein:
Fig. 1 is a perspective view of an exemplary embodiment of a product line for producing pouches according to a second aspect of the invention.
Fig. 2 is a perspective view of an exemplary embodiment of a dosing unit of the product line.
Fig. 3 is a perspective view of another exemplary embodiment of the dosing unit of the product line.
Fig. 4 is a perspective view of another exemplary embodiment of a product line for producing pouches.

### Detailed description of preferred embodiments of the invention

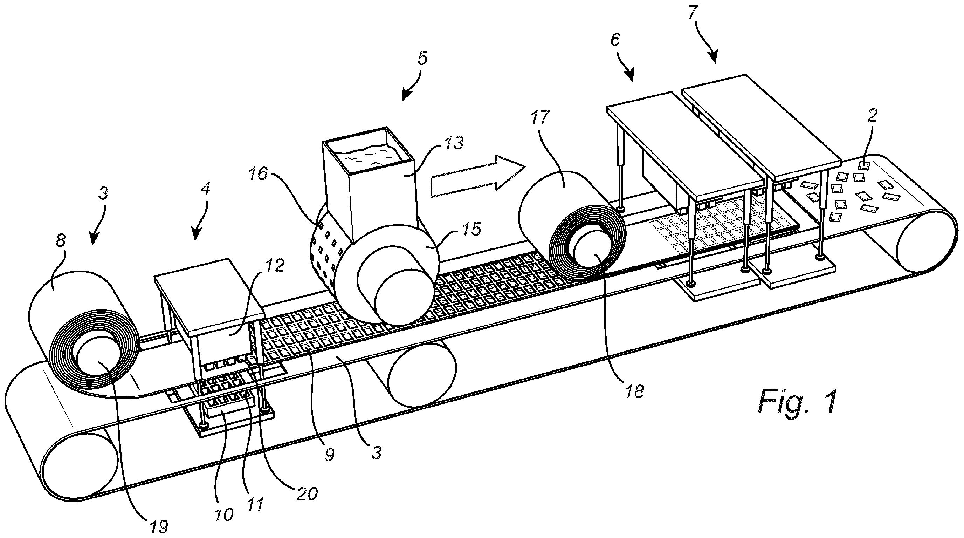

Fig. 1 illustrates an exemplary embodiment of a product line 1 for producing pouches 2. The product line 1 comprises a supply unit 3, a deformation unit 4, a dosing unit 5, a sealing unit 6 and a cutting unit 7.

In this specific embodiment, the supply unit 3 consists of a moving band which is used to transport a first layer 8 of material along the production line 1. The first layer of material is being supplied from a roll 19 of material. Naturally, other solutions are also possible. The first layer 8 of material can for example be arranged on a roll at one side of the product line 1 and be pulled by some sort of drive unit from the other side of the product line 1.

The first layer 8 of material is supplied through the deformation unit 4 by means of the moving band. In the deformation unit 4, the first layer 8 of material is deformed to create recesses 9 therein. The deformation unit 4 comprises a first part 10 having recesses 11, and a second part 12 having protrusions 20. The first part 10 is arranged below the moving band and the second part 12 is arranged above the moving band. This way, the protrusions 12 can be arranged to engage with the recesses 11 of the first part 10.

The first layer 8 of material is further transported through the dosing unit 5 by means of the moving band. The dosing unit 5 is arranged downstream of the deformation unit 4 and adapted to provide a formulation in the recesses 9 of the first layer 8 of material. The dosing unit 5 comprises a reservoir 13 for holding the formulation. The reservoir 13 is connected to a roll 15 which is equipped with recesses 16 adapted to receive a specified amount of the formulation from the reservoir 13. The roll 15 is rotatably arranged in relation to the reservoir 13.

The first layer 8 of material is further transported through the sealing unit 6 by means of the moving band. Into the sealing unit 6 is also a second layer 17 of the material transported by means of the moving band. The second layer 17 of material is being supplied from a roll 18 of material. The sealing unit 6 is arranged downstream of the dosing unit 5 and arranged to seal the first layer 8 of material and the second layer 17 of material together in a plurality of sealing sections.

The sealed first layer 8 and second layer 17 of material is finally transported through the cutting unit 7 by means of the moving band. The cutting unit 7 is arranged downstream of the sealing unit and arranged cut the first layer 8 of material and the second layer 17 of material in the sealing sections, thereby forming a plurality of pouches 2.

Naturally, further layers, such as protective layers, can be added to the first 8 and second 17 layer of material at any suitable place in the production line 1.

The product line 1 can be adapted in width to process any suitable material size. In the embodiment described above, the first layer 8 and the second layer 17 of material has a width that allows four pouches to be manufactured simultaneously in the different units of the production line 1. Naturally, without a theoretical end of the first and second layer 8, 17 of material, that number of pouches manufactured simultaneously can be varied in a number of different ways.

The formulation provided in the pouches 2 is chosen from the group consisting of dry powder, a wet powder and a liquid solution dosed into an absorbing material. The formulation comprises nicotine or synthetic or cannabis derived cannabinoids, cannabinoid acids, terpenoids, flavonoids and mixtures thereof.

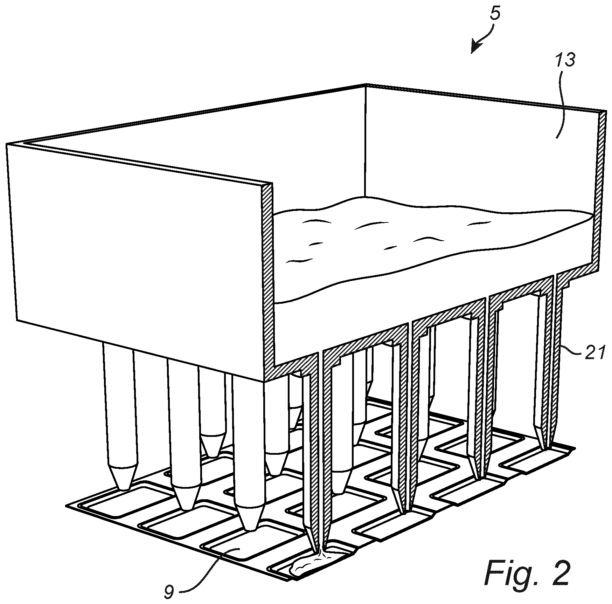

In Fig. 2, another exemplary embodiment of the dosing unit 5 is illustrated. Here, the dosing unit 5 consists of a reservoir 13 holding the formulation and supply means 21 projecting downwardly from the reservoir 13 to supply the formulation into the recesses 9 of the first layer 8 of material.

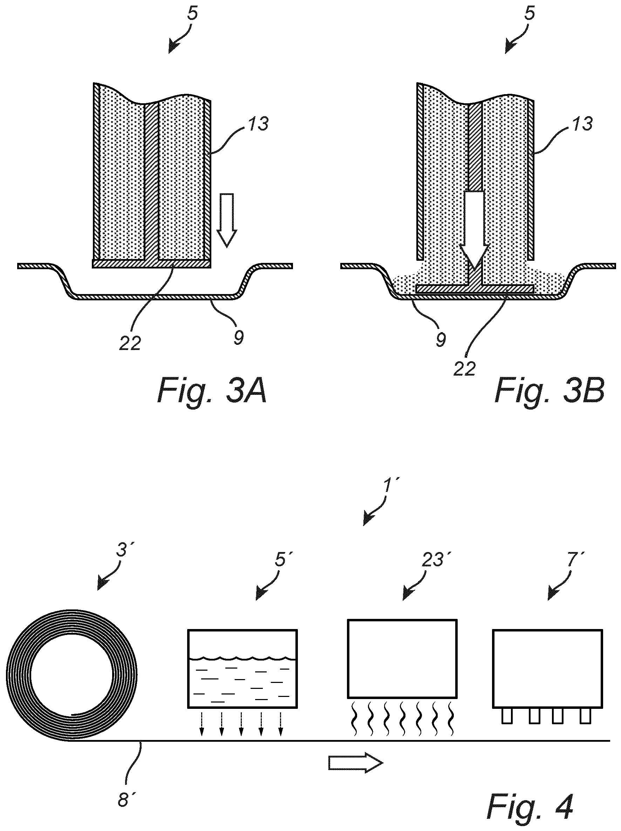

Fig. 3 illustrates yet another exemplary embodiment of the dosing unit 5. In this embodiment, the dosing unit 5 comprises a reservoir 13 for holding the formulation. The reservoir 13 is movable in a vertical direction in relation to the production line 1 and has an openable bottom portion 22 for releasing a specified amount of the formulation into a recess 9 of the first layer 8 of material.

When the product line 1 according to the embodiment described above is in operation, the first layer 8 of material is transported from start to finish by means of the moving band.

The first layer 8 of material first reaches the deforming unit 4 which creates recesses 9 in the same by pressing the first part 12 and the second part 10 of the deforming unit 4 together, thereby clamping the first layer 8 of material between the engaging protrusions 20 and recesses 11.

The first layer 8 of material is thereafter transported to the dosing unit 5 in which the recesses 9 are provided with formulation from the reservoir 13. The roll 15 rotates continuously while gravity makes sure to provide formulation in the recesses 16 of the roll. The formulation is thereafter, by means of gravity, passed on into the recesses 9 of the first ayer 8 of material passing through the dosing unit.

The first layer 8 of material is thereafter transported into the sealing unit 6 by means of the moving band. Into the sealing unit 6 is also a second layer 17 of the material transported by means of the moving band. The second layer 17 of material is being supplied from a roll 18 of material in a similar manner to that of the first layer 8 of material. Note that one or more additional layers, such as a protective layer or the like, can also be added at this stage.

In the sealing unit 6, the first layer 8 and the second layer 17 of the material are sealed together in sealing sections by means of heat sealing or ultrasonic welding. The number of sealing sections naturally depends on the width of the layers 8 ,17. Both longitudinal and transversal sealing sections are normally provided.

Thereafter, as a final step, the sealed first 8 and second layer 17 of material are cut in the cutting unit 7 in order to create the finished pouch product 2. The material is preferably cut in the center of the sealing sections.

In Fig. 4, another exemplary embodiment of a product line 1' for producing pouches is illustrated. This product line 1' comprises a supply unit 3' transporting a first layer 8' of material along the production line 1'. The supply unit 3' can basically consist of a roll of material which is pulled or somehow transported along the product line 1'. The first layer 8' of material is preferably some sort of absorbing material or a non-woven material.

The product line 1' further comprises a dosing unit 5' arranged downstream of the supply unit 3' or the roll of material. The dosing unit 5' comprises a reservoir 13' containing the formulation, preferably in liquid form. The formulation is supplied onto the first layer 8' of material by means of the dosing unit 5'. The formulation can basically be supplied from the dosing unit 5' from openings or supply means, such as projections, arranged in the bottom portion of the reservoir 13'.

The product line 1' further comprises a heating unit 23' arranged downstream of the dosing unit 5' which is used to provide heat onto the first layer 8' of material in order for the formulation to dry into the first layer 8' of material.

Finally, the product line 1' comprises a cutting unit 7' arranged downstream of the heating unit 23'. The cutting unit 7' is arranged to cut the first layer 8' of material, thereby forming at least one pouch or pouch like product.

Optionally, a second layer of material can be introduced along the product line 1', for example between the heating unit 23' and the cutting unit 7'. As an alternative, or in addition, further layers of material can be introduced along the product line 1'. Such layers of material can for example consist of a protective layer or the like.

The product line 1' can be adapted in width to process any suitable material size. In the embodiment described above, the first layer 8' of material only has a width which allows one pouches to be manufactured at the time in the different units of the production line 1'. Naturally, without a theoretical end of the first 8', and optionally the second layer of material, that number of pouches manufactured simultaneously can be varied in a number of different ways.

The skilled person realizes that a number of modifications of the embodiments described herein are possible without departing from the scope of the invention, which is defined in the appended claims.

For instance, further units and/or other components may be arranged along the product line for different reasons

## Claims

1. A method for producing pouches (2), comprising
feeding a first layer (8) of material into a deformation unit (4),
deforming the first layer (8) of material in the deformation unit (4), thereby creating recesses (9) in the first layer (8) of material,
feeding the first layer (8) of material into a dosing unit (5),
providing a formulation in each recess (9) of the first layer (8) of material,
feeding a second layer (17) of material onto the first layer (8) of material,
sealing the first layer (8) of material and the second layer (17) of material together in at least one sealing section, and
cutting the first layer (8) of material and the second layer (17) of material in the at least one sealing section, thereby forming at least one pouch (2).

2. The method according to claim 1, wherein the formulation provided in the pouches (2) is chosen from the group consisting of dry powder, a wet powder and a liquid solution dosed into an absorbing material.

3. The method according to claim 1 or 2, wherein the formulation comprises nicotine.

4. The method according to any one of the preceding claims, wherein the formulation comprises synthetic or cannabis derived cannabinoids, cannabinoid acids, terpenoids, flavonoids and mixtures thereof.

5. The method according to any one of the preceding claims, wherein the step of feeding the first layer (8) of material into a deformation unit (4) comprises placing a first layer (8) of material from a roll onto a moving band.

6. The method according to any one of the preceding claims, wherein the step of sealing the first layer (8) of material and the second layer (17) of material together in at least one sealing section comprises heat sealing or ultrasonic welding.

7. The method according to any one of the preceding claims, wherein the step of cutting the first layer (8) of material and the second layer (17) of material in the at least one sealing section comprises stamping out a plurality of pouches (2) in one single operation.

8. The method according to any one of the preceding claims, further comprising the step of feeding a protective layer onto the first layer (8) of material and/or the second layer (17) of material before sealing the first layer (8) of material and the second layer (17) of material together.

9. A production line (1) for producing pouches (2), comprising
a supply unit (3) transporting a first layer (8) of material along the production line (1), the first layer (8) of material optionally being supplied from a roll of material,
a deformation unit (4), through which the first layer (8) of material travels, adapted to deform the first layer (8) of material to create at least one recess (9) therein,
a dosing unit (5) arranged downstream of the deformation unit (4), through which the first layer (8) of material travels, adapted to provide a formulation in the at least one recess (9) of the first layer (8) of material,
a sealing unit (6) arranged downstream of the dosing unit (5), through which the first layer (8) of material travels, and through which a second layer (17) of material is supplied, the sealing unit (6) being adapted to seal the first layer (8) of material and the second layer (17) of material together in at least one sealing section, and
a cutting unit (7) arranged downstream of the sealing unit (6), through which the sealed first layer (8) and second layer (17) of material travels, adapted to cut the first layer (8) of material and the second layer (17) of material in the at least one sealing section, thereby forming at least one pouch (2).

10. The production line (1) according to claim 8, wherein the supply unit (3) comprises a moving band onto which the first layer (8) of material is placed to travel along the production line (1).

11. The production line (1) according to claim 8 or 9, wherein the deformation unit (4) comprises a first part (10) having recesses (11), and a second part (12) having protrusions (20) adapted to engage with the recesses (11) of the first part (10), the second part (12) being arranged above the first part (10).

12. The production line (1) according to any one of claims 8-10, wherein the dosing unit (5) comprises a reservoir (13) for holding the formulation connected to a roll (15) equipped with recesses (16) adapted to receive a specified amount of the formulation, the roll (15) being rotatably arranged in relation to the reservoir (13).

13. The production line (1) according to any one of claims 8-10, wherein the dosing unit (5) comprises a reservoir (13) for holding the formulation and being movable in a vertical direction in relation to the production line (1), the reservoir (13) having openable bottom portion (22) for releasing a specified amount of the formulation into a recess (9) of the first layer (8) of material.

14. The production line (1) according to any one of claims 8-12, wherein the formulation provided in the pouches (2) is chosen from the group consisting of dry powder, a wet powder and a liquid solution dosed into an absorbing material.

15. The production line (1) according to any one of claims 8-13, wherein the formulation comprises nicotine or synthetic or cannabis derived cannabinoids, cannabinoid acids, terpenoids, flavonoids and mixtures thereof.
